# EUROPEAN PATENT APPLICATION

(11) **EP 4 686 934 A1**
(43) Date of publication of application: **04.02.2026**
(21) Application number: 24306299.9
(22) Date of filing: 31.07.2024
(51) Int. Cl.: G01N 22/00, G01N 33/00

(54) **RADIOFREQUENCY SENSING SYSTEM FOR SELECTIVE GAS AND VAPOR DETECTION**

(71) Applicant: UNIVERSITE DE BORDEAUX, 33000 Bordeaux (FR); Centre National de la Recherche Scientifique, 75016 Paris (FR); INSTITUT POLYTECHNIQUE DE BORDEAUX, 33400 Talence (FR); Université Gustave Eiffel, 77454 Marne la Vallee (FR); Université de Limoges, 87032 Limoges (FR)
(72) Inventor: HALLIL, Hamida, 33800 BORDEAUX (FR); NGOUNE, Bernard Bobby, 33800 BORDEAUX (FR); DEJOUS, Corinne, 33360 QUINSAC (FR); LEBENTAL, Bérengère, 91720 BUNO-BONNEVAUX (FR); PERRIN, GUILLAUME, 75013 PARIS (FR); DUMON, Marine, 93160 NOISY LE GRAND (FR); BILA, Stéphane, 87430 VERNEUIL SUR VIENNE (FR); CLOUTET, Eric, 33130 BEGLES (FR)
(74) Representative: Plasseraud IP

(57) **Abstract**

Gas radiofrequency sensing system and method for sensing at least one gas present in a mixture of gases and vapors, and environment interferents, comprise a plurality of radiofrequency "RF" passive sensors (100, 101, 102, 103, 104) and a trained machine learning model to predict from the trained machine learning model at least one first quantitative value corresponding to a concentration of one target environmental gas from said mixture of gases and vapors and at least one second quantitative value corresponding to an interferent parameter corresponding to operating conditions of the gas sensing system.

## Description

### Technical Field

This disclosure relates to the field of air quality monitoring. More precisely, the present disclosure relates to a radiofrequency gas sensing system for sensing one or more gases in a mixture of gases and vapors and one or more environmental parameters.

In the present disclosure, by radiofrequency senor, what is meant is a sensor able to operate in frequency range comprised between 3 kHz and 300 GHz.

### Background Art

The significance of air quality has transcended its previous status as a trivial environmental concern, capturing considerable attention in recent years. This newfound emphasis can be attributed primarily to the rapid pace of urbanization, intensified industrial activities, and the proliferation of transportation systems that release substantial volumes of toxic gases, profoundly impacting public health. Consequently, there has been a notable uptick in the gas sensor market, reflecting a growing awareness of the urgent need for comprehensive air quality monitoring.

Atmospheric pollution involves several particles and a complex multi-component gaseous system which may include carbon monoxide resulting in smog formation (CO), carbon dioxide (CO₂), nitrogen oxides (NOₓ), Sulphur oxides (SOₓ), and other volatile organic compounds (VOCs), ozone (Os), and fine particles where concentration is usually detected in the rage of parts per million (ppm) for health and environmental applications. The presence of these components in the atmosphere may have short- and long-term health impacts on both humans and animals, and may cause health issues like heart diseases, respiratory diseases and cancers. Therefore, it is essential to monitor their presence in real time in the atmosphere to mitigate these adverse effects.

Microwave transduction-based sensors with their high operating frequency (300 MHz - 300 GHz) and robustness are of great interest in air quality monitoring since they are suitable for non-invasive, non-contact measurements, they can be fabricated on flexible substrates and additive technologies and can be widely deployed for many applications.

Microwave sensors have emerged as a promising technology in the air quality monitoring, since they can offer non-intrusive and highly sensitive measurement over traditional methods. These sensors can detect a wide range of pollutants, including particulate matter, gases, and volatile organic compounds, with high precision and accuracy.

Additionally, they can be fabricated by additive technologies and can be widely deployed for many applications.

Furthermore, microwave transduction-bases sensors operate at high operating frequency and can be integrated easily in wireless communication technologies with reduced energy consumption and data analytics platforms, allowing for data collection, analysis, and interpretation.

Two technologies are mainly used by microwave transduction-based sensors. One approach consists in measuring physical properties like wave propagation, wave speed (physical gas sensors). Another approach consists in measuring the interaction of the target gas with the sensitive material (chemical gas sensors).

In a first approach, microwave sensors work without sensitive material, the interaction of the microwave with the target species directly affect the electromagnetic wave propagation, thereby causing a change in the sensor's response.

In a second approach, the microwave sensors detect, and measure gases based on the physical-chemical interactions. They are composed of two parts: a sensitive material and a transducer. The sensitive material, when exposed to the surrounding atmosphere, interacts with target gas. The interaction induces a change in one or more physicochemical properties of the material, such as electrical conductivity, permittivity which in turn converted into an electrical signal or a measurable output by the transducer, resulting in the detection of a specific gas.

For gas sensing, the transducers are commonly associated to a variety of gas sensitive materials like metal oxides, carbon nanotubes (CNTs), graphene, transition Metal Dichalcogenides and polymers.

The sensors can be classified into two categories: active and passive. Active sensors require the use of a battery source for their operation, leading to increase energy consumption. In contrast, passive sensors do not necessitate an energy source to operate. Such passive sensor is coupled to a control unit which applies an interrogation signal to an input of the passive sensor and able to read an output signal corresponding to the change of the interrogation signal based on the variation of the physicochemical properties of the sensitive material. Such system may be for example a vector network analyzer (VNA).

The present disclosure relates in particularly to the use of a differential passive microwave sensors combined with polymer sensitive material to detect gases and determine their concentration.

However, when a gas is part of a multi-component system, using one material to detect a specific gas is difficult because of the cross-sensitivities of the materials to the various component gases of the mixture. Other gas components in the mixture gases will affect the signal of the gas detectors leading to failed detection towards the specific gas through complex mixture conditions.

In addition, when the sensors operate in uncontrolled real-life conditions, the variation in the sensor signal can be affected by the fluctuation of the ambient humidity level and the temperature, and other interferent parameters, making gas detection complex and difficult. Thus, it is necessary to correct the recognition of gases by using additional humidity and temperature sensors.

Thus, the deployability of microwave sensors in environmental monitoring is limited by the drift behavior of sensors overtime, humidity level, temperature variation and the lack of selectivity.

One object of the present disclosure is to provide a gas sensor array system for monitoring air quality by detecting a wide variety of gas molecules in real-world outdoor environments, easily to be performed, and having low energy consumption.

Another object of the present disclosure is to increase the sensitivity, specificity and selectivity of the sensors and provide a gas sensor array system having a strong capability to discriminate and measure one or more specific gases even in a complex mixture and under interferences from ambient humidity and temperature.

### Summary

This disclosure improves the situation.

The subject matter disclosed in detail below is directed to a gas radiofrequency sensing system for sensing at least one gas or one vapor present in a mixture of gases and vapors, the gas sensing system comprising:
- a plurality of radiofrequency "RF" passive sensors, each of the RF sensors being configured to generate at least one signal within a microwave frequency range;
- a calculating unit comprising a memory and a processor connected to the memory, said memory storing a trained machine learning model;
- said trained machine learning model being preliminary trained on a calibration model establishing a calibration relationship between each of the signal from the plurality of RF passive sensors and a plurality of reference environmental parameters, said reference environmental parameters including at least one reference gas and at least one reference interferent parameter;
- said processor communicating with the plurality of RF passive sensors and configured to:
   ∘ acquire a plurality of signals from the plurality of radiofrequency passive sensors;
   ∘ input in the trained machine learning model the plurality of signals;
   ∘ analyze the plurality of signals by the trained machine learning model;
   ∘ predict from the trained machine learning model at least one first quantitative value corresponding to a concentration of one target environmental gas or vapor from said mixture of gases and vapors and at least one second quantitative value corresponding to an interferent parameter corresponding to operating conditions of the gas sensing system.

The combination of an array of RF passive sensors and the use of a trained machine learning model offers an accurate specific, selective and sensitive gas detection, even though in outdoor conditions with the presence of interferents.

Thanks to the trained machine learning model and the data generated by an array of RF passive sensors, it is possible to construct a multiparametric model that relate sensors responses to different variables of interest. This specific combination enables a dynamic in-situ calibration, e.g. in a realistic environment.

The trained machine learning model mitigates cross-sensitivity and environmental interferences, such as humidity and temperature through calibration model suppression.

The RF passive sensors allow a low energy consumption. Indeed, there is no need external energy and the interrogation signal may be transmitted from a VNA or a wireless unit.

The following features, can be optionally implemented, separately or in combination one with the others:

In one or more embodiments, the gas radiofrequency sensing system may comprise a plurality of reference sensors different from the RF passive sensors, each reference sensor being configured to generate a reference signal corresponding to a reference environmental parameter.

In one or more embodiment, the trained machine learning model may be based on a relationship between each of the signal from the plurality of RF passive sensors, a set of input environmental variables to be predicted, said set of input environmental variables including at least one interferent parameter and at least one concentration of one target gas or vapor from said mixture of gases and vapors.

In one or more embodiments, at least one RF passive sensor among the plurality of RF passive sensors may comprise a first resonator and a second resonator, said second resonator being coated with a sensitive layer that interacts with a specific gas to produce a variation in the electromagnetic properties of the second resonator, each RF passive sensor being configured to generate a first signal called reference signal produced by the first resonator and a second signal called measurement signal related to said variation produced by said second resonator.

In one or more embodiments, the plurality of signals input in the trained machine learning model may correspond to a differential signal from the difference between the reference signal and the measurement signal.

In one or more embodiments, said sensitive layer may comprise:
- a combination of multiple materials selected from carbon nanotubes, graphene, and reduced graphene oxide to modulate the conductivity of the sensitive layer;
- a combination of one or more materials selected from carbon nanotubes, graphene, and reduced graphene oxide with nanoparticles to enhance the sensitivity and selectivity of the sensitive layer;
- nanoparticles or nanowires of metal oxides, such as ZnO, TiO₂, and graphene oxide, incorporated into the sensitive layer to improve conductivity and sensitivity of the sensitive layer;
- metallic nanoparticles, including Au, Ag, and Pd, integrated into the sensitive layer to enhance detection capabilities.
- a polymer layer chosen in the group comprising: polyethyleneimine (Com HBPEI), Polyethyleneimine silane (HBPEI-Silane), linear polyethyleneimine COPh (LPEI-COPh), linear polyethyleneimine CH2-Ph (LPEI-CH2-Ph).

In one or more embodiments, the plurality of RF passive sensors may comprise an array of polyethyleneimine based sensor (I31 COM HBPEI-based sensor), linear polyethyleneimine based sensor (I23 LPEI-COPh-based sensor), polyethyleneimine silane-based sensor (I33 HBPEI-Silane-based sensor), linear polyethyleneimine CH2-Ph based sensor (I34 LPEI-CH2-Ph based sensor

In one or more embodiments, the I31 COM HBPEI-based sensor may be used as a humidity sensor and the I23 LPEI-COPh-based sensor is used as a temperature sensor.

In one or more embodiments, the plurality of signals may comprise a frequency information, a phase information and a magnitude information.

In one or more embodiments, the at least one interferent parameter may include at least one of a temperature, humidity, wind speed, pressure.

In one or more embodiments, the at least one target environmental gases and vapors may be chosen in the group comprising: NO₂, O₃, CO, VOC, CO₂, NO, H₂S, SO₂, NH₃, O₂, Cl₂, H₂, liquefied petroleum gas, Acetylene, polycyclic aromatic hydrocarbons (PAHs), aromatic hydrocarbons: benzene, toluene, xylene, ethyl benzene; halogenated hydrocarbons: chloroethylene, trichloroethylene; propane, butane, methane methyl chloride; formaldehyde, toluene, acetone, isopropyl alcohol; pesticides: chlordane, DDT; plasticizers: phthalates ;Refrigerant gases: hydrofluorocarbons, chlorofluorocarbons, hydrochlorofluorocarbons.

In one or more embodiments, each of RF passive sensor may be configured to generate a signal within a microwave frequency range between 0.3 and 300 GHz, preferably between 0.5 GHz and 10 GHz.

In one or more embodiments, the sensing system may further comprise a reader unit configured to generate and transmit a measurement interrogation signal and a reference interrogation signal to each of the gas sensors, receive the measurement signal and the reference signal from each of the gas sensors and transmit the measurement signal and the reference signal to said calculating unit.

In one or more embodiments, the machine learning model may be a K-Nearest Neighbors, a Random Forest, a Gaussian Process Regression models algorithm.

In another aspect, it is proposed a method for operating a sensing system as defined above, said method comprise a measurement phase comprising:
- generating a plurality of signals within a microwave frequency range by each of the RF passive sensors;
- acquiring the plurality of signals by the processor;
- inputting in the trained machine learning model the plurality of signals;
- analyzing the plurality of signals by the trained machine learning model;
- predicting from the trained machine learning model at least one first quantitative value corresponding to a concentration of one target gas or vapor from said mixture of gases and vapors and at least one second quantitative value corresponding to an interferent parameter corresponding to operating conditions of the gas sensing system

In one embodiment, the method may further comprise a training phase performed before the measurement phase, said training phase comprising:
- acquiring a plurality of signals from each of the radiofrequency passive sensors;
- acquiring a plurality of reference environmental parameters from a plurality of reference sensors, said reference environmental parameters including at least one reference gas and at least one reference interferent parameter;
- inputting in the machine learning model the plurality of signals and the plurality of reference environmental parameters;
- training said machine learning model on a calibration model establishing a calibration relationship between each of the signal from the plurality of RF passive sensors and the plurality of reference environmental parameters;
- storing said trained machine learning model in the memory.

### Brief Description of Drawings

Other features, details and advantages will be shown in the following detailed description and on the figures, on which:
**Fig. 1**

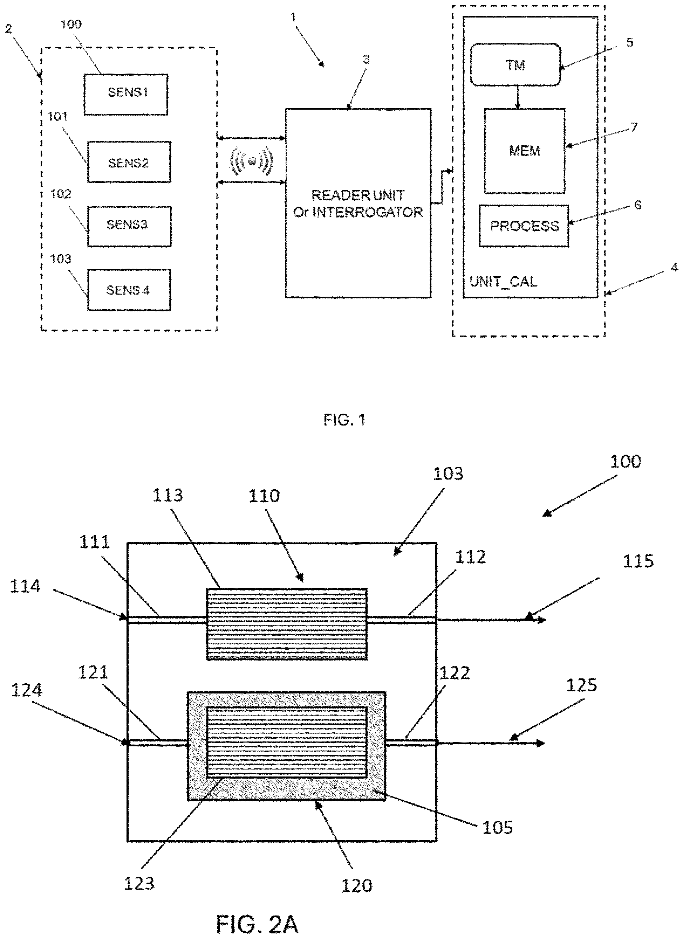

   [Fig. 1] Figure 1 is a schematic view of a gas sensing system according to an embodiment.
**Fig. 2**
   [Fig. 2A] Figure 2A is a schematic top view of a RF gas sensor according to an embodiment of the present disclosure.

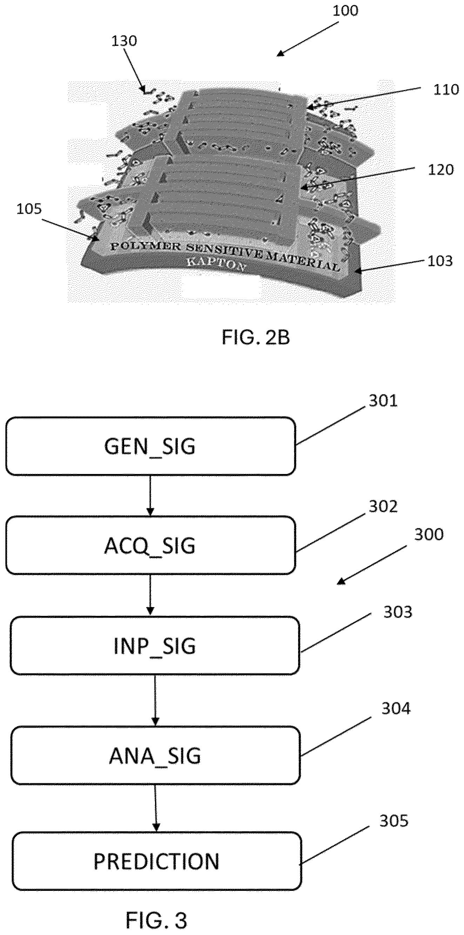

[Fig. 2B] Figure 2B is a schematic view of the RF gas sensor of Figure 2A showing two microstrip interdigitated resonators printed on a flexible Kapton substrate, one of the resonators being coated with a sensitive polymer layer, while the other remains uncoated.
**Fig. 3**
   [Fig. 3] Figure 3 is a flowchart showing steps of a method for performing the measurement phase using the gas sensing system of Figure 1.
**Fig. 4**

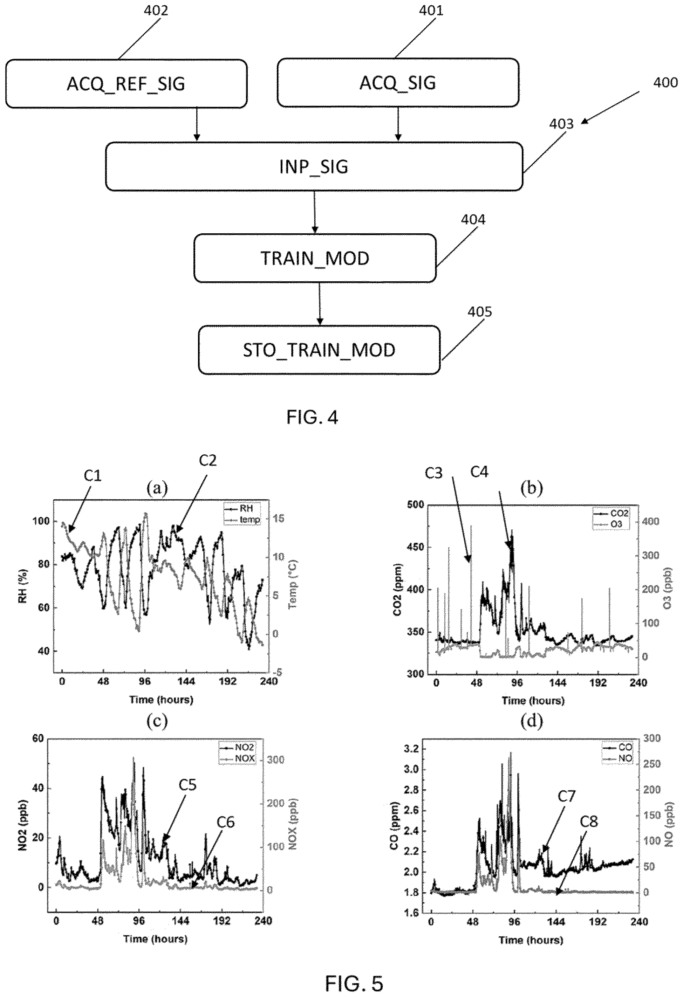

   [Fig. 4] Figure 4 is a flowchart showing steps of the training phase.
**Fig. 5**
   [Fig. 5] Figure 5 are graphs showing environmental parameters measured in Sense-city which offers outdoor environmental conditions by the corresponding environmental sensors, for example the Vaisala WXT536 weather station to track temperature and humidity, the Envea CO12e for CO and CO₂ , the Envea AC32e for NO and NO₂, and the Envea O342e for O₃); (a) RH(%) and temperature (C°), (b) CO₂ (ppm) and Os (ppb), (c) NOₓ (ppb) and NO₂ (ppb) and (d) CO(ppm) and NO (ppb).
**Fig. 6**

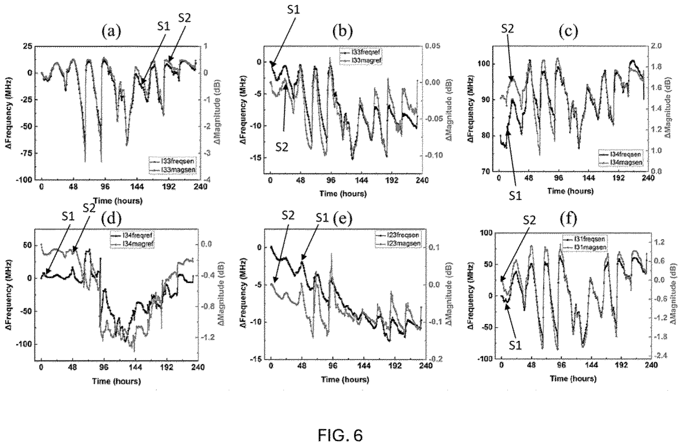

   [Fig. 6] Figure 6 represents frequency and magnitude responses measured by a sensor array: (a) represents responses measured by the sensitive resonator of the sensor I33 (HBPEI Silane 1:0.5 - based sensor); (b) represents responses measured by the reference resonator of the resl33 (HBPEI Silane 1:0.5 - based sensor); (c) represents responses measured by the sensitive resonator of the sensor I34 (LPEI-CH₂-Ph-based sensor) (d) represents responses measured by the reference resonator of the sensor I34 (LPEI-CH₂-Ph-based sensor); (e) represents response measured by the sensitive resonator of the sensor I23 LPEI-CoPh; (f) represents response measure measured by the sensitive resonator of the sensor I131 COM HBPEI-based sensitive resonator.

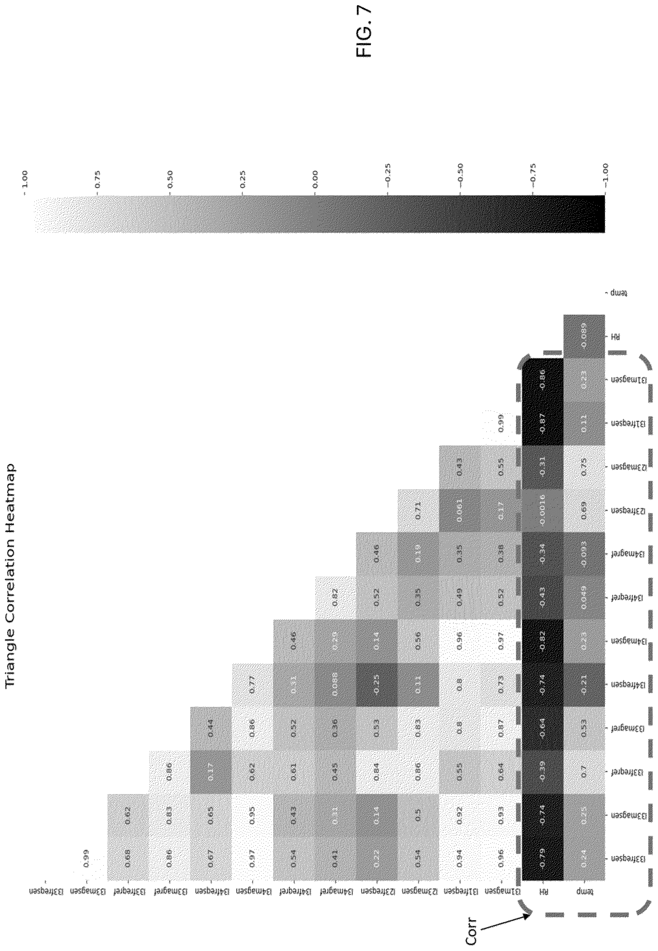

[Fig. 7] Figure 7 represents the correlation heatmap of the differential frequency and magnitude responses measured by a sensor array consisting of the sensitive and reference resonators of sensor I33 HBPEI Silane, I34 LPEI-Ch₂Ph, and the sensitive resonator of sensors I23 LPPEI-CoPh and I31 COM HBPEI, with the reference environmental parameters measured in Sense-city by the reference sensors.

### Description of Embodiments

To facilitate understanding, identical reference numerals have been used, where possible, to designate identical elements that are common to the figures. It is contemplated that elements disclosed in one embodiment may be beneficially utilized on other embodiments without specific recitation.

The present disclosure relates to a microwave gas sensing system and method for outdoor and indoor gases sensing, specifically a wireless sensor array combined with a trained machine learning model to detect one or more gases and vapors of interest within an outdoor and indoor environment. The gases and vapors of interest may include, but is not limited to, nitrogen dioxide (NO₂), ozone (Os), carbon monoxide (CO), VOC, CO₂, NO, H₂S, SO₂, NH₃, O₂, Cl₂, Hz, hydrocarbures aromatiques polycycliques (HAP) liquefied petroleum gas, Acetylene, polycyclic aromatic hydrocarbons (PAHs), aromatic hydrocarbons: benzene, toluene, xylene, ethyl benzene; halogenated hydrocarbons: chloroethylene, trichloroethylene; propane, butane, methane methyl chloride; formaldehyde, toluene, acetone, isopropyl alcohol; pesticides: chlordane, DDT; plasticizers: phthalates ;Refrigerant gases: hydrofluorocarbons, chlorofluorocarbons, hydrochlorofluorocarbons.

In the present disclosure, an "array" or "network" of sensors is meant at least two different sensors that are spatially separated and placed in a measuring environment, the measuring environment being an outdoor or indoor environment.

In the present disclosure, a microwave sensor or radiofrequency (RF) passive sensor refers to a sensor that may operate at frequencies in a range from about 1 GHz to about 10 GHz.

In the present disclosure, a representative measurement environment may be an indoor or an outdoor environment. An indoor environment may include any indoor infrastructure, such as habitats, schools, health care facilities, industrial building, cabin air etc. An outdoor environment may include any urban outdoor infrastructure, such as parking, highways, street etc.

The microwave gas sensor is a passive sensor that is not powered by an external power source.

The microwave gas sensor utilizes a microstrip interdigitated resonator integrated with a sensitive material for gas sensing. The sensitive material for gas sensing may be a polymer sensitive material. In one example, an interdigitated resonator may be combined to a polymer sensitive material, but other microwave topologies and other type of sensitive materials may be used for the detection of target gas. The sensing principle is based on the variation of the sensitive material's permittivity upon the absorption of the target gas. Consequently, the permittivity and the impedance of the resonator will then vary, and change the sensor electrical response, frequency, magnitude and phase. This passive sensor is coupled to a reader unit or interrogator, such as a Vector Network Analyzer (VNA) that applies an interrogation signal to the sensor and receives the electrical response of the sensor. The variation of the dielectric properties of the sensing material upon exposure to the target gases causes changes in the electrical response. Based on the electrical response, the reader unit may measure the shift in the resonance frequency, its magnitude and phase. These parameters may be input and analyzed by the machine learning model to determine a quantitative value corresponding to the concentration of a gas of interest present in the environment.

This microwave gas passive sensor system allows real-time detection of gases at room temperature over a wide concentration range and is adapted for the world of the Internet of Things since it operates already in the radio frequency signal transmission range.

In addition, unlike other conventional microwave gas sensor, the present disclosure provides an array of microwave sensors to collect a plurality of signals and the help of a machine learning model to analyze the plurality of signals to predict a quantitative value relative to at least one of a gas from the gases and at least one environmental value relative to at least one of an environmental value parameter, such as the humidity and/or temperature.

In addition, in the prior art, the calibration model is often based on an individual sensor deployed in known environmental conditions, for instance laboratory conditions. Therefore, alterations in environmental conditions, time-dependent drift, cross-sensitivities in the sensor responses, can negatively impact the accuracy of measurements. The present disclosure provides a machine learning model that is trained on a plurality of signals from a plurality of microwave gas passive sensors and environmental parameters from a plurality of reference sensors. During this training phase, the signals from the sensors and the reference environmental parameters are input to a processing unit in order to optimize parameters used in the machine learning model. Then, it is possible to conduct accurate measurements in outdoor environments without filtering, e.g. in presence of interference parameters, such as ambient humidity, ambient temperature, ambient pressure, wind speed.

Therefore, the gas sensing system, based on an array of cross-sensitive gas sensors combined with a trained machine learning model, allows the detection and an accurate quantitative estimation of one or more specific gases and vapors even in complex mixture conditions, despite the cross interferences from the reactions between different vapor and the sensor array, with reduced cost and easy access.

In the present disclosure, a reference sensor refers to a standard sensor difference from the RF passive sensor configured to acquire reference environmental parameters of the outdoor and indoor environment. The reference environment parameters include the reference interferent parameters. An interferent parameter includes, but is not limited to temperature, humidity, pressure or wind speed, etc.

In the present disclosure, an interferent parameter refers to an undesired environmental parameter that can affects the accuracy and precision of measurement with the sensors.

It is now referred to figure 1 which depicts a radiofrequency gas sensing system 1 according to an embodiment.

The system 1 comprises a plurality of RF gas passive sensors forming a gas sensor network 2 and a control system. The plurality of RF gas passive sensors is controlled by the control system to detect one or more gases of interest present in the environment and to predict at least one environmental parameter.

The plurality of RF gas passive sensors may be implanted or incorporated in different supports for real-time monitoring of one or more gases of interest present in the environment and one or more environmental parameters, such as ambient humidity. Non-limiting examples of implanting or incorporation of the sensors may include industrial indoors infrastructure, urban outdoors infrastructure, urban indoors infrastructure, roads, buildings, bridges etc.

The control system may for instance include a reader unit 3 and a computer or calculating unit 4. In this example, the reader unit 3 is used for interrogating the sensors and acquiring signals therefrom, while the computer 4 is used for controlling the reader unit 3, analyzing the signals acquired by the reader unit 3 by a trained machine learning model and predicting one or more concentration of a gas of interest and one or more environmental parameters. In a variant, a single electronic device could fulfill all the functionalities of the control unit 3 and the computer 4.

The plurality of RF passive sensors is located remotely from the control system. The reader unit 3 may be communicatively coupled to each of the plurality of RF passive sensors via bi-directional communication links. The bi-directional communication links may be based on one or more wireless protocols such as Bluetooth Low Energy, Bluetooth, WiFi, LoRaWan. The bidirectional communication links may be configured to transmit and receive signal.

The reader unit 3 is configured to transmit an interrogation electrical signal at a frequency between 500 MHz and 300 GHz, more specifically between 1 and 10 GHz. For instance, the interrogation signal may a sinusoidal waveform having an amplitude and a frequency. The reader unit 3 may adjust the frequency of the waveform at a resonant frequency of the sensor. In other words, the sensors transmit the signal to the reader unit 3 at frequency comprising in the RF range. The reader unit 3 is configured to receive an impedance response of the sensor in response to the interrogation signal. In accordance with another example, the reader unit 3 may be a VNA and the sensors are connected to the VNA.

The calculating unit 4 comprises a memory 7 and a processor 6 connected to the memory. The memory 7 is a storage device configured to store signal acquired from the plurality of RF passive sensors and environmental parameters acquired from the environmental sensors. In addition, the memory 7 may store a trained machine learning model (TM) 5 trained to predict the concentration of one or more gases and vapors in the environment and one or more environmental parameters, for example temperature, humidity, pressure.

In accordance with one embodiment, the trained machine learning model may operate locally on the sensors.

In accordance with one embodiment, the trained machine learning model may be run in the processor.

In another embodiment, the trained machine learning model may be run in a cloud-based platform.

The machine learning model 5 is preliminary trained on a calibration model which establishes a calibration relationship between each of the signal from the plurality of RF sensors and a plurality of reference environmental parameters. The reference environmental parameters include at least one reference gas and at least one interferent reference parameter.

According to an embodiment, the plurality of reference interferent parameters includes at least one of a temperature, humidity, wind speed, pressure.

According to an embodiment, the at least one reference gas or vapor measured is chosen in the group comprising: nitrogen dioxide (NO₂), ozone (Os), carbon monoxide (CO), VOC, CO₂, NO, H₂S, SO₂, NH₃, O₂, Cl₂, H₂, liquefied petroleum gas, Acetylene, polycyclic aromatic hydrocarbons (PAHs), aromatic hydrocarbons: benzene, toluene, xylene, ethyl benzene; halogenated hydrocarbons: chloroethylene, trichloroethylene; propane, butane, methane methyl chloride; formaldehyde, toluene, acetone, isopropyl alcohol; pesticides: chlordane, DDT; plasticizers: phthalates ;Refrigerant gases: hydrofluorocarbons, chlorofluorocarbons, hydrochlorofluorocarbons.

The reference environmental parameters are measured by a plurality of reference sensors different from the RF passive sensors.

The contents of the memory 7 may be accessed by the processor 6.

In accordance with one embodiment, the processor 6 is configured to analyze the plurality of signals by the machine learning model 5 and predict from the trained machine learning model a first quantitative value corresponding to a concentration of at least one target gas from said mixture of gases and a second quantitative value corresponding to at least one environmental parameter corresponding to operating conditions of the gas sensing system.

In accordance with one embodiment, the raw signal from each of RF gas passive sensors is converted to a numeric signal. The numeric signal can then be sent to the processor 6 for analysis.

The computer 4 may also comprise a communication interface able to communicate with the results of the measurement to a communication device. The communication device may be a smartphone, a tablet or a computer. More precisely, the communication device may comprise a dedicated application through which the results are communicated to a user.

The communication interface may be a wired interface, for example using USB ports, or a radio interface using aWi-Fi, Bluetooth^{®}, LORA^{®}, SigFox^{®} or NBIoT network. In another embodiment, it can also communicate using a 2G, 3G, 4G or 5G network.

### RF sensors design

The architecture of a RF passive sensor according to an example will be described in detail below with reference to Figure 2.

In the present disclosure, a RF gas passive sensor may be an electromagnetic (EM) transduction passive sensor. By "EM transduction passive sensor" is meant a passive sensor whose measurement of the value of the physical quantity is based on a variation in the distribution of the electromagnetic field in the sensor as a function of the variation in the physical quantity, resulting in a change in the interrogation signal applied at the sensor input. RF sensors design developed in the present disclosure obey to the same rules as passive elements: filters, resonators, antennas, and another topology.

Radiofrequencies (RF) are a range of electromagnetic frequencies that fall within the electromagnetic spectrum. RF waves have frequencies ranging from about 3 kilohertz (kHz) to 300 gigahertz (GHz). These frequencies are higher than those of audio frequencies but lower than those of infrared radiation and visible light and allow an easy integration of the sensors network in a wireless communication system.

With reference to Figures 2A and 2B, the RF gas passive sensor 100 comprises a first resonator 110 and a second resonator 120 on a substrate 103. A resonator is a device or structure that exhibits resonance at certain frequencies, meaning it has a natural tendency to oscillate strongly at specific frequencies.

The first resonator 110 without any sensitive layer is called reference resonator and configured to produce a reference signal. The second resonator 120 is coated with a sensitive layer 105 that interacts with a specific gas 130 to produce a variation in the electromagnetic properties of the second resonator. This second RF passive sensor is called measurement resonator and configured to produce a measurement signal. The operating principle of the sensitive resonator 120 is based on the variation of the electrical properties, in particular permittivity, conductivity and/or permeability, due to the presence of the target gas 130 on the sensitive layer. The reference resonator 110 compensates the variation not related to the sensitive material. Thus, the two resonators allow a differential detection to compensate the variation not related to the sensitive material. Consequently, during the measurement phase, each of the RF gas sensors generate a differential and the signal input in the machine learning model corresponds to a differential signal from the difference between the reference signal and the measurement signal.

Planar transmission lines may be interesting for sensing applications due to their ease of fabrication, small size, low cost, ability to be flexible and wireless etc. Unlike waveguides and coaxial lines, planar transmission lines are two-dimensional components, consisting of conductors on dielectric substrates. The planar transmission line can be either homogeneous or inhomogeneous depending on whether the conductor is surrounded by a uniform medium or not.

According to an embodiment, the resonators may be based on planar technologies (microstrip line and coplanar wave guides) with a specific resonator geometry. The resonator of the present disclosure may be specifically fabricated to obtain a resonance frequency around 3.3 GHz so that the RF sensor may be able to communicate wirelessly in microwave range. For example, the RF sensor may be integrated in an Internet of Things (loT) network. IoT refers to the network of interconnected devices embedded with sensors, software, and other technologies that enable them to collect and exchange data over the internet. These devices can range from everyday objects like household appliances and wearable devices to industrial machines and infrastructure components. IoT technology allows for seamless communication and coordination between devices, leading to automation, data-driven insights, and improved efficiency in various domains such as home automation, healthcare, transportation, agriculture, and manufacturing.

Microwaves are a type of electromagnetic radiation characterized by their relatively short wavelengths and high frequencies. Microwaves are commonly used in various technological applications due to their unique properties. Microwaves are extensively utilized in telecommunications, including satellite communication, microwave radio links, and radar systems. They fall within the range of radiofrequencies: microwaves have frequencies ranging from about 300 megahertz (MHz) to 300 gigahertz (GHz), corresponding to wavelengths between 1 millimeter and 1 meter. These frequencies place microwaves between radio waves and infrared radiation on the electromagnetic spectrum. In the present disclosure, the sensors are configured to operate at an operating frequency between 0.5 GHz and 10 GHz, preferably at 3.3 GHz.

Therefore, the technology of microwave sensors offers distinct advantages over alternative technologies, such as their cost-effectiveness, low energy consumption, passive nature, non-intrusiveness, ability to be flexible (conformable), and wireless readout capability.

For instance, the designed microwave sensor 110, 120 may comprise a resonator made of 200 µm wide, 20 µm thick copper lines printed on a flexible Kapton substrate 103 with a thickness of 100 µm.

The resonator presents an interdigitated structure as illustrated in Figure 2B which is chosen to maximize the surface electric field density, allowing enhanced sensitivity to the changes in the dielectric properties of the sensitive materials at the surface when interacting with the target gas. The length of the interdigitated lines is half a wavelength.

In accordance with one embodiment, the designed sensor may be a five fingered microstrip interdigitated resonator made of 200 µm wide, 20 µm thick copper lines printed on the Kapton substrate. The length of the interdigitated fingers may be half a wavelength, for example 30 mm. To achieve an operating frequency of 3 GHz, a Kapton substrate with a thickness of 100 µm and a dielectric constant of 3.5 are used.

For the purpose of miniaturization, the interdigitated electrodes are bent to maintain the same electric length while reducing the overall structure's length as shown in Figure 2A. The bending is done in a manner that the juxtaposed fingers are in opposite directions to allow an optimal coupling between the input port and the output port of the resonator.

The design of the RF passive sensors is not limited the example of Figures 2A and 2B. The RF passive sensors may comprise volumic and planar RF topologies with either filter, resonator or antenna geometries for example, split ring resonator, double split ring resonator, coupled line, microstrip spiral resonator, half wave and quarter wave resonators.

In the example of Figures 2A and 2B, the first resonator 110 comprises a first interdigitated structure 113 and the second resonator 120 comprises a second interdigitated structure 123. Each interdigitated structure 113, 123 comprises an input port or an input feed line 111, 121 and an output port 112, 122. The control unit 3 comprises a plurality of ports configured to be connected respectively to the input 114, 124 of the input port 111, 121 to apply an interrogation signal. The control unit 3 comprises a plurality of ports configured to be connected respectively to the output 115, 125 of the out port 112, 126 to receive a signal generated by the resonators in response to the applied interrogation signal.

The RF passive sensor is configured to receive a reference interrogation signal at the input 114 and a measurement interrogation signal at the input 124 and to generate a reference signal at the output 115 and a measurement signal at the output 125.

The measurement signal and the reference signal each comprise, for example a frequency information, a phase information and a magnitude information. Due to the presence of the sensitive material, there is a shift between the measurement signal and the reference signal, for instance a shift in the phase, in the magnitude or in the frequency.

Kapton may be specifically chosen as the substrate for the microstrip gas sensor due to its several unique characteristics. It may offer high thermal mechanical strength and good flexibility making it suitable for flexible applications.

Moreover, Kapton's resistance to a wide range chemicals and its low outgassing properties may further enhance its suitability for gas sensing purposes. Additionally, with exceptional thermal stability, Kapton can endure extreme temperature conditions from -260 °C to 400 °C, making it an ideal choice for designing robust sensors.

A double-sided copper-clad Kapton laminate, with a thickness of 100 µm, may be chosen for sensor design and fabrication. This material may be well-suited for multilayer flex and rigid-flex applications, due to its high hydrophobicity and excellent thermal resistance. To prevent oxidation, the copper-clad substrate may be treated with an immersion coating of electroless nickel and gold.

These RF passive sensors can be further enhanced in terms of sensitivity and selectivity by functionalizing sensitive materials 105. Then, microwave gas sensors use the interaction of microwaves with the gaseous species absorbed by gas sensitive materials.

Sensitive material 105 may serve as a fundamental component in gas and vapor sensors. Its selection may be crucial, as it may interact directly with the gas sensing transducer. To qualify as a good sensing material, several criteria must be met, such as affordability and ability to be functionalized. For flexible sensors, the sensitive material must be able to withstand mechanical deformation while maintaining consistent sensing performance during bending or stretching.

Sensitive materials may be metal oxides for advantages such as a low cost and a high sensitivity to several gases. For example, metal oxides sensitive materials may be TiO₂, Hematite (Fe2O₃), CuO₂, SnO₂, WO₃, or MoO₃.

Alternatively, sensitive materials may be Carbon NanoTubes (CNTs) which have a high surface-to-volume ratio enhancing sensitivity and a room temperature operation. For example, CNTs sensitive materials may be Single-Wall CNTs, Multi-Wall CNTs (MWCNTs), or poly (3,4-ethylenedioxythiophen) polystyrene sulfonate - MWCNTs.

Alternatively, sensitive materials may be graphene which have a high surface area increasing sensitivity and a broad range of detectable gases. For example, graphene sensitive materials may be reduced graphene oxide (rGO).

Alternatively, sensitive materials may be conducting polymers which have a room temperature operation, a good sensitivity to some, organic compounds and may be functionalizable and tunable. Conducting polymers such as Polyanaline (PANI), Polypyrrole (PPy), Polythiophene (PTh), Poly(3,4-ethylenedioxythiphene) (PEDOT) along with their derivatives, may be utilized in gas sensing.

The sensitive materials may comprise:
- a combination of multiple materials selected from carbon nanotubes, graphene, and reduced graphene oxide to modulate the conductivity of the sensitive layer;
- a combination of one or more materials selected from carbon nanotubes, graphene, and reduced graphene oxide with nanoparticles to enhance the sensitivity and selectivity of the sensitive layer;
- nanoparticles or nanowires of metal oxides, such as ZnO, TiO₂, and graphene oxide, incorporated into the sensitive layer to improve conductivity and sensitivity of the sensitive layer;
- metallic nanoparticles, including Au, Ag, and Pd, integrated into the sensitive layer to enhance detection capabilities of the sensitive layer.

Alternatively, sensitive materials may be non-conducting polymers which have a low cost, flexibility, stability, a minimal electric interference, and may be functionalized with various molecules for sensing target gas. They may be polyethyleneimine (PEI) nanofibers or polydimethylsiloxane (PDMS).

In accordance with one embodiment, PEI may be used as the base polymer. PEI is an amorphous polymer with a highly branched structure. This amorphous nature may give PEI unique properties, such as high solubility and good adhesion to various substrates. Gas sensing abilities of PEI materials primarily may rely on the presence of amine groups, whether there are primary, secondary or tertiary amino groups. A commercial hyper-Branched PEI (COM HBPEI) may be used as the base product to be functionalized with various molecules resulting in distinct sensitive polymers. 1,2-epoxybutane polyethyleneimine (EB-PEI), polyethyleneimine silane (HBPEI Silane), linear polyethyleneimine COPh (LPEI-COPh), and linear polyethyleneimine CH₂-Ph (LPEI-CH₂-Ph) may be synthesized and used as sensitive materials for the microwave sensors.

The sensitive polymers, e.g. commercial HBPEI, EB-PEI, HBPEI Silane, LPEI-COPh, CH₂-Ph (LPEI-CH₂ -Ph) are deposited on the resonators using a spin coating process.

In accordance with one embodiment, prior the deposition, the substrates undergo a prebaking process for 4 hours at 120°C in an air oven. The deposition is achieved by laminating the flexible Kapton substrate onto a double-face adhesive placed over a glass slide. To protect the reference resonator, an adhesive tape is applied before the deposition process. Following the deposition, the coated sensors are baked for reticulation using different methods, such as hotplate under air, air oven, nitrogen oven.

In the example of Figure 1, the system comprises four RF gaz sensors. A first sensor 100 (SENS1) comprises a sensitive resonator coated with a commercial PEI material, called I31 COM HBPEI based sensor. A second sensor 102 (SENS2) comprises a sensitive resonator coated with a HBPEI Silane material, called I33 HBPEI Silane based sensor. A third sensor 103 (SENS3) comprises a sensitive resonator coated with a LPEI-COPh material, called I23 LPEI-COPh based sensor. A fourth sensor 104 (SENS4) comprises a sensitive resonator coated with a LPEI-CH₂-Ph material, called I134 LPEI-CH₂-Ph based sensor. The number of gas sensors is not limited to four. The system may comprise two or more than two RF passive sensors.

Table 1 provides a summary of the four sensors with the various polymer layers deposited. The table includes information about the thickness of the deposited layers and the sensitive surface area corresponding to the area surrounding the interdigitated structure with surface.

**[Tab. 1]**

| Sensor name | Sensitive material | Thickness | Sensitive surface |
|---|---|---|---|
| I31 COM HBPEI | Commercial HBPEI | 1.2 µm | 21×20 mm² |
| I33 HBPEI Silane | HBPEI Silane 1:0.5 | 1.2 - 1.3 µm | 17×22 mm² |
| I23 LPEI-COPh | LPEI-COph | 1.2 µm | 16.5×26 mm² |
| I134 LPEI-CH₂-Ph | LPEI-CH_{2 - Ph} | 1.1 µm | 17×20 mm² |

The thickness of the sensitive layer may be comprised between 1 µm and 1.5 µm. The thickness of the sensing layer is a crucial parameter that influences the behavior of sensors. Thicker films may enhance sensitivity to analyte concentration due to an increased number of reaction sites. However, thicker films lead to longer response time as gas molecules diffusive over greater distances. These trade-offs result in an optimal thickness for improved sensitivity and response time. An optimal thickness is based on the resonance frequency variation and changes in the dielectric properties.

The signals received by the control unit 3 are transmitted to the processor 6.

In one embodiment, the processor 6 is configured to:
- acquire the plurality of signals from the plurality of radiofrequency passive sensors;
- input in the trained machine learning model the plurality of signals;
- analyze the plurality of signals by the trained machine learning model;
- predict from the trained machine learning model a first quantitative value corresponding to a concentration of at least one gas from said mixture of gases and a second quantitative value corresponding to at least one interferent parameter corresponding to operating conditions of the gas sensing system.

According to an embodiment, the plurality of interferent parameters includes at least one of a temperature, humidity, wind speed, pressure.

According to an embodiment, the at least one target gas predicted by the gas sensing system is chosen in the group comprising: nitrogen dioxide (NO₂), ozone (Os), carbon monoxide (CO), VOC, CO₂, NO, H₂S, SO₂, NH₃, O₂, Cl₂, H₂, liquefied petroleum gas, Acetylene, polycyclic aromatic hydrocarbons (PAHs), aromatic hydrocarbons: benzene, toluene, xylene, ethyl benzene; halogenated hydrocarbons: chloroethylene, trichloroethylene; propane, butane, methane methyl chloride; formaldehyde, toluene, acetone, isopropyl alcohol; pesticides: chlordane, DDT; plasticizers: phthalates ; refrigerant gases: hydrofluorocarbons, chlorofluorocarbons, hydrochlorofluorocarbons.

In accordance with one embodiment, before using the fabricated RF passive sensors under various targets gases in indoor or outdoor conditions, their response relative to the environmental parameters is evaluated. In particular, the sensing characteristics of the RF passive sensors to humidity and temperature are evaluated in laboratory conditions.

The laboratory setup for the characterization of gas sensor under gas includes a test cell in which is placed the sensor to tested, a calibration gas generator to produce different gas concentration, a humidity generator that produces RH ranging from 1 to 90%, a climatic chamber used to generate humidity from 30 to 90 % RH in air at different temperatures from 20 - 90°C, a VNA controlled by a computer to extract the sensor Sᵢⱼ parameters, and a commercial temperature and humidity sensor. The calibration has generator that produces known gas concentration to calibrate sensors.

The responses of each sensor have been studied under laboratory conditions. In particular, the sensing characteristics of each sensorto humidity and temperature have been studied. The effect of humidity of each sensor is evaluated in a range of 0-90% relative humidity at 25°C.The effect of temperature of each sensor is evaluated at constant relative humidity.

The measurement results show the shift in the resonance frequency when the sensors are exposed to relative humidity "RH" from 30 to 90% RH. The sensor I31 exhibits heightened sensitivity to RH. The sensors I23 and I34 show diminished sensitivity to RH. The sensor I31 is a good comprise as humidity sensor. When exposed to outdoor conditions, RH is a prevalent interferent parameter, the sensors I33, I23 and I34 with reduced sensitivity to RH, are suitable candidate sensors for sensing target gases.

The sensing characteristic of the sensors to temperature are evaluated at a constant relative humidity. The sensor I31 exhibits the highest temperature sensitivity, followed by the sensor I23, the sensor I33 and the sensor I34.

The outdoor conditions are emulated by a platform, called sense-city, a large-scale facility replicating the main outdoor environment such as roads, housing, and vegetation. Air quality monitoring in the sense-city offers two setting: open outdoor measurements and a controlled environment inside a large 20x20x10 cm climate chamber. The entire sense-city platform is monitored by an array of environmental sensors providing reference measurements for training the machine learning model. In one example, the environmental sensors may include commercial environmental sensors for NO, NO₂, O₃, CO and CO₂. A weather station may be used to track temperature and humidity, rain and wind speed and direction.

### Machine learning model

In the present disclosure, the machine learning model is trained to predict the concentration of one or more gases and one or more interferent environmental parameters.

The trained machine learning model is based on a calibration model establishing a relationship between each of the RF passive sensor output signals denoted y and a set of input variables denoted x to be predicted. The x variables to be predicted include one or more interferent environmental parameters and one or more gases present in the environment and the y signals include sensor differential signals. For example, the sensor differential signal may be the differential frequency, called freqdiff and magnitude, called magdif. For example, the environmental parameters to be predicted include the temperature, the pressure, the humidity and the wind speed.

In the example of Figure 1, the sensing system comprises four RF passive sensors and the calibration model establishes a relationship between each of the four sensors output signals, denoted yᵢ, 1 ≤ i ≤ 4, and the input variables denoted x to be predicted.

In accordance with one embodiment, the inputs x may include a plurality of environmental variables including relative humidity, temperature and a plurality of gases including O₃, NOₓ, CO, CO₂.

In one embodiment, the machine learning model for calibration may be based on a Bayesian framework. It amounts to assuming that the values (here gas concentration) to be estimated and the measurement errors can be modelled by random quantities. In another embodiment, the model may be used without Bayesian framework.

In a variant, the Bayesian calibration is based on non-parametric inference using kernel density techniques. This approach consists of modeling complex, non -linear relationships between the sensor's responses and inputs without assuming a specific form.

In another variant, the Bayesian calibration is parametric and based on adapted forms of Generalized Linear Regression or Gaussian Process Regression accounting for uncertainties on reference measurements, for error in the calibration model, for measured and unmeasured factors of influence, and for correlation between references measurements.

The machine learning model may be a K-Nearest Neighbors model algorithm, a Random Forest model or a Gaussian Process Regression model. The memory 7 may store at least one of four models and the processor 6 is configured to select one of the models to analyze the signals from the RF passive sensors and to predict the concentration of one or more gases and one or more environmental parameters.

In accordance with one embodiment, the model may be used while accounting for uncertainties on sensors outputs, and/or on target gas concentration, and/or on interferent parameters.

The machine learning model may be preliminary trained on a calibration model establishing a calibration relationship between each of the signals from the plurality of RF passive sensors and a plurality of reference environmental parameters, said reference environmental parameters including at least one reference gas concentration and at least one reference interferent r parameter.

In accordance with one embodiment, the machine learning model may be trained before to predict the concentration of one or more gases, and two major interferents parameters which are humidity and temperature in outdoor environment on a reference dataset.

The calibration relationship may be then used to predict the value of humidity and the concentration of gas with only the knowledge of the calibration relationship over the training reference dataset and the outputs of the sensors.

In accordance with one embodiment, the machine learning calibration model may be trained under indoor conditions, for example laboratory conditions. The laboratory calibration could be transferred to outdoor to reduce calibration duration of the plurality of RF sensors to the plurality of environmental parameters and gaseous species. This solution can facilitate the transition from controlled laboratory measurement to more complex outdoors measurements without involving recalibrating the sensors under the new environmental conditions. Calibration transfer can be made at level of the sensor response, the calibration model and the calibration prediction results that can be based respectively on response standardization, model update and model output standardization.

In the response standardization, the response from a secondary sensor is arithmetically modified to resemble the response produced by a primary sensor.

In the model update, the calibration model developed with the primary sensor data is retrained with few samples from the secondary sensor data.

In the model output standardization, the calibration model developed from the primary sensor is used as it with the secondary sensor response to get the target value.

In accordance with another embodiment, the machine learning calibration model may be trained directly under outdoor conditions, for example sense-city conditions as described above.

The machine learning model may be trained in outdoor environmental conditions in a purpose to take account of environmental parameters that may affect the sensor's response, by modifying its sensitivity to the target gas. The system is calibrated directly under environmental conditions. In another embodiment, the machine learning model may be prior trained in indoor environmental conditions.

With reference to Figure 3, an example of use of the system of Figure 1 will now be described.

In this example, an array of the four passive sensors listed in Table 1 are used. The array of four gas passive sensors comprises: I31 COM HP=BPEI (only sensitive resonator), I23 LPEI-COPh (only sensitive resonator), I33 HBPEI Silane (both reference and sensitive resonators), and I34 LPEI-CH₂-Ph-bsesd sensors (both reference and sensitive resonators). Therefore, the array provides 6 frequency outputs and 6 magnitude outputs. For the I33 and I34 sensors, both the sensitive and reference resonators are recorded, resulting in 8 output signals. For the I23 and I31 sensors, the sensitive resonators are recorded, resulting in 4 output signals. The four sensors are deployed in the SENSE-CITY platform as described above.

In the present disclosure, a sensor array is an arrangement of several distinct sensors working together to achieve higher accuracy in a complex environment. The sensor array combined with a multi-output calibration model, allows to enhance not only the selectivity and the sensitivity but also the precision in predicting RH, temperature and concentrations of specific gases and vapors. In addition, the multiparameter measurements allow to act as backup in case of a sensor failure.

At step 301, called "GEN_SIG", each of four RF passive sensors generates a measurement signal within microwave frequency range, for instance at 3.3 GHz.

At step 302, called "ACQ_SIG", the processor acquires the signals. This step may be implemented continuously or periodically.

At step 303, called "INP_SIG", the signals are input in the trained machine learning model.

At step 304, called "ANA_SIG", the trained machine learning model analyzes the signals.

At step 305, called "PREDICTION", the processor predicts from the trained machine learning model a first quantitative value corresponding to a concentration of at least one gas or vapor from a mixture of gases and vapors and a second quantitative value corresponding to one or more environmental parameters corresponding to operating conditions of the gas sensing system.

According to an embodiment, the combination of an array of RF passive sensors and a trained machine learning model allows the prediction of humidity, temperature and ozone levels in uncontrolled outdoor environments.

Figure 4 illustrates an exemplary training phase 400 of the machine learning model to produce a trained machine learning model (TM) for processing the acquired signals transmitted from the RF passive sensors.

At step 401, called "ACQ_SIG", each of four sensors generates a measurement signal within microwave frequency range, for instance at 3.3 GHz.

At step 402, called "ACQ_REF_SIG", a plurality of reference sensors which are not RF sensors acquires a plurality of reference environmental parameters. For example, the reference environmental parameters may correspond to ambient temperature, humidity, and concentrations of NO₂, O₃, CO₂, NO₂, CO etc.

Both steps 401 and 402 may be performed simultaneously or subsequently.

At step 403, called "INP_SIG", the signals from the sensors and the reference parameters from the reference sensors are input in the machine learning model. Thus, in the present disclosure, the calibration uses the sensor signals as variable with the environmental parameters to ameliorate the prediction capabilities of the system.

The model can be trained with several combinations of available environmental parameters and a combination of the RF sensors responses.

For example, the combinations of variables may be one of the following combinations:
- humidity + NO₂,
- humidity + NO₂ + temperature,
- humidity + NO₂ + temperature + I33 Sensor response,
- humidity + NO₂ + I33 Sensor response,
- NO₂ + I31 Sensor response,
- NO₂ + I31 Sensor response + I23 Sensor response,
- NO₂ + I31 Sensor response + I23 Sensor response + I33 Sensor response.

In accordance with one embodiment, the inventors note that using the sensor array consisting of I31 COM HBPEI-based sensor, I23 LPEI-COPh-based sensor and the uncoated resonators could enable to replace the direct use of the environmental variables like RH and temperature. Indeed, the COM HBPEI-based sensor can serve as reliable RH sensor, while the LPEI-COPh and the uncoated resonators can serve as reliable temperature sensors. Thus, the COM HBPEI-based sensor can be used as humidity sensor, offering advantage in terms of mass production on flexible substrate and a good sensitivity in frequency and magnitude. When the array of sensors exposed to outdoor conditions, similar sensors but coated with functionalized with different materials, for example HBPEI Silane and LPEI based sensors, with their reduced sensitivity to humidity, appears suitable candidate sensor for reliable outdoor air quality monitoring.

At step 404, called "TRAIN_MOD", the machine learning model is trained on a calibration model establishing a calibration relationship between each of the signal from the plurality of RF sensors and a plurality of environmental parameters to adjust parameters of the trained machine learning model.

The steps 401 to 404 may be repeated for a plurality of measurement cycles.

At step 405, called "STO_TRAIN_MOD", the trained machine learning model is stored in the memory.

Figure 5 shows the environmental parameters measured by the standard sensors placed in the SENSE-CITY platform.

The graph (a) shows measured relative humidity "RH" (%) and temperature (°C) over time, represented respectively by the curves C2 and C1. The graph (b) shows measured CO₂ (ppm) C4 and Os (ppb) C3 overtime, represented respectively by the curves C4 and C3. The graph (c) shows measured NO₂ (ppb) C5 and NOₓ (ppb) C6 overtime, represented respectively by the curves C5 and C6. The graph (d) shows measured CO (ppm) C7 and NO(ppb) C8 over time, represented respectively by the curves C7 and C8.

Figure 6 shows differential frequency response represented by the curve S1 and differential magnitude response represented by the curve S1 for the four RF passive sensors during their outdoor deployment.

The graph (a) represents responses measured by the sensitive resonator of the sensor I33 (HBPEI Silane 1:0.5 - bases sensor).

The graph (b)represents responses measured by the reference resonator of the resl33 (HBPEI Silane 1:0.5 - bases sensor).

The graph (c) represents responses measured by the sensitive resonator of the sensor I34 (LPEI-CH₂-Ph-based sensor).

The graph (d) represents responses measured by the reference resonator of the sensor I34 (LPEI-CH2-Ph-based sensor).

The graph (e) represents response measured by the sensitive resonator of the sensor I23 LPEI-Coph.

The graph (f) represents response measure by the sensitive resonator of the sensor I131 COM HBPEI-bases sensitive resonator.

Figure 7 represents a correlation heatmap between RF passive sensor outputs and environmental parameters. Correlation values of 1 or -1 denote perfect positive or negative linear correlation between pairs of parameters, and zero value signifies no linear correlation.

This correlation is based on 2 environmental parameters as inputs (temperature, relative humidity) and 12 sensors outputs, corresponding respectively to the output signals measured by the sensitive resonator of I31 sensor (I31magsen,I31freqsen), the output signals measured by the sensitive resonator of I23 sensor (I23magsen, I23freqsen), the output signals measured by both reference and sensitive resonators of I34 sensor (I34freqref, I34magref, I34magsen, I34fresen), the output signals measured by both reference and sensitive resonators of I33 sensor (sensol33magref, I33freqref, I33magsen, I33fresen).

The inventors note that a strong correlation exists between each sensor frequency and magnitude response. The frequency and magnitude response of the sensitive and reference resonators of the sensors I31, I33 and I34 are highly correlated while showing minimum correlation with the reference resonator, and sensitive resonator of sensor I23. The response of the reference resonators on their side are highly correlated to the sensitive resonator response of sensor I23. This is in line with the fact that the COM HBPEI (I31), HBPEI Silane 1:0.5 (I33) and LPEI-CH2-Ph (I34)-based sensors are highly sensitive to relative humidity "RH" while the uncoated resonators (reference resonator) and LPEI-COPh-based sensor (I23) are more sensitive to the temperature as shown by the correlation plot, named Corr in Figure 7.

These results show that relative humidity and temperature are the most influencing environmental parameters on the sensor array response.

Table 2 shows the results of the KNN and RF models performance on ozone prediction using several combination of environmental variables and sensors array responses as variables.

**[Tab 2]**

| Variable | **KNN** | | **RF** | |
|---|---|---|---|---|
| | **MAE (ppb)** | **MAPE (%)** | **MAE (ppb)** | **MAPE (%)** |
| **RH+NO₂** | **11.04** | **13.18** | **11.45** | **14.23** |
| RH+NO₂+temp | 12.83 | 9.68 | 10.76 | 9.93 |
| **RH+NO₂+I33SE** | **10.28** | **12.61** | **11.01** | **12.9** |
| RH+NO₂+temp+I33SE | 13.32 | 11.7 | 10.95 | 10.92 |
| **I31SE+NO₂** | **9.76** | **12.26** | **10.1** | **12.54** |
| I31SE+I23SE+NO₂ | 12.28 | 12.43 | 9.87 | 12.15 |
| **I31SE+NO₂+I33SE** | **9.71** | **12.21** | **10.01** | **12.43** |
| I31SE+I23SE+NO₂+I33SE | 11.97 | 12.5 | 9.79 | 12.20 |

The model performance is measured by Mean absolute error (MAE) and Mean absolute percentage error (MAPE).

MAE is the average absolute differences between the predicted and actual values. It quantifies the error on the predictions made by the calibration model.

MAPE is the average of the absolute percentage differences between the predicted and the actual values. Unlike MAE, MAPE is a relative measure of error and is scale-independent, making it useful to compare predictions across different scales.

The results show the importance of knowing NO₂ to predict ozone. Using the environmental variables, such as RH and NO₂ enables prediction of ozone with a good MAE and MAPE (11.04 ppb, and 13.18% for KNN and 11.45 ppb and 14.23% for RF). Adding the magnitude response measured by the sensitive resonator of I33 sensor, corresponding to the combination RH+NO₂+I33SE, improves the MAE and MAPE (10.28 ppb, and 12.61% for KNN and 11.01 ppb and 12.9% for RF) affirming the secondary sensitivity of the HBPEI Silane 1:0.5 to ozone.

Using the magnitude response measure by the sensitive resonator of I31 sensor (I31SE) instead for RH and the magnitude response measured by the sensitive resonator of I23 sensor (I23SE) instead of temperature with the I33SE sensor response, corresponding to the combination I31SE + I23SE+ NO₂ +I33SE, increases the ozone prediction capabilities of the models KNN and RF.

The result shows that using the sensor array consisting of I31 sensor (COM HBPEI-based sensor), I23 sensor (LPEI-COPh-based sensor), and the reference resonators of I33 and I34 can enable to replace the direct use of the environmental variable like RH and temperature. The COM HBPEI-based sensor can be used as reliable relative humidity "RH" sensor and the LPEI-COPh-based sensor can be used as reliable temperature sensor.

These results show that the gas sensing system and method of the present disclosure may be used as a reliable, and robust tool for monitoring the quality air. The data from the system may be integrated into a central control system that uses machine learning model or prediction model to predict pollution levels, taking into account traffic data, weather conditions. The use of flexible, highfrequency microwave passive sensors, may allow to enhance the detection capabilities, making the system more efficient than traditional sensors. The system of the present disclosure may be used to monitor CO₂ levels in industries, indicating inefficiencies or leaks.

## Claims

1. Gas radiofrequency sensing system (1) for sensing at least one gas or one vapor present in a mixture of gases and vapors, the gas sensing system comprising:
- a plurality of radiofrequency "RF" passive sensors (100, 101, 102, 103, 104), each of the RF passive sensors being configured to generate at least one signal within a microwave frequency range;
- a calculating unit (4) comprising a memory (7) and a processor (6) connected to the memory, said memory storing a trained machine learning model (5);
- said trained machine learning model being preliminary trained on a calibration model establishing a calibration relationship between each of the signal from the plurality of RF passive sensors and a plurality of reference environmental parameters, said reference environmental parameters including at least one reference gas and at least one reference interferent parameter;
- said processor (6) communicating with the plurality of RF passive sensors and configured to:
∘ acquire a plurality of signals from the plurality of RF passive sensors;
∘ input in the trained machine learning model the plurality of signals;
∘ analyze the plurality of signals by the trained machine learning model;
∘ predict from the trained machine learning model at least one first quantitative value corresponding to a concentration of one target gas or vapor from said mixture of gases and vapors and at least one second quantitative value corresponding to an interferent parameter corresponding to operating conditions of the gas RF sensing system.

2. Gas radiofrequency sensing system according to claim 1, further comprising a plurality of reference sensors different from the RF passive sensors, each reference sensor being configured to generate a reference signal corresponding to a reference environmental parameter.

3. Gas radiofrequency sensing system according to claim 1 or 2, wherein the trained machine learning model is based on a relationship between each of the signal from the plurality of RF passive sensors, a set of input environmental variables to be predicted, said set of input environmental variables including at least one interferent parameter and at least one concentration of one target gas or vapor from said mixture of gases and vapors.

4. Gas radiofrequency sensing system according to any of claims 1 to 3, wherein at least one RF passive sensor among the plurality of RF passive sensors comprises a first resonator (110) and a second resonator (120), said second resonator being coated with a sensitive layer (105) that interacts with a specific gas to produce a variation in the electromagnetic properties of the second resonator (120), each RF passive sensor being configured to generate a first signal called reference signal produced by the first resonator (110) and a second signal called measurement signal related to said variation produced by said second resonator (120).

5. Gas radiofrequency sensing system according to 4, wherein the plurality of signals input in the trained machine learning model corresponds to a differential signal from the difference between the reference signal and the measurement signal.

6. Gas radiofrequency sensing system according to any of claims 4 to 5, wherein said sensitive layer (105) comprises:
- a combination of multiple materials selected from a group comprising: carbon nanotubes, graphene, and reduced graphene oxide to modulate the conductivity of the sensitive layer (105);
- a combination of one or more materials selected from a group comprising: carbon nanotubes, graphene, and reduced graphene oxide with nanoparticles to enhance the sensitivity and selectivity of the sensitive layer (105);
- nanoparticles or nanowires of metal oxides, such as ZnO, TiO₂, and graphene oxide, incorporated into the sensitive layer to improve conductivity and sensitivity of the sensitive layer (105);
- metallic nanoparticles, including Au, Ag, and Pd, integrated into the sensitive layer (105) to enhance detection capabilities;
- a polymer layer chosen in the group comprising: polyethyleneimine (Com HBPEI), Polyethyleneimine silane (HBPEI-Silane), linear polyethyleneimine COPh (LPEI-COPh), linear polyethyleneimine CH₂-Ph (LPEI-CH₂-Ph).

7. Gas radiofrequency sensing system according to any of claims 1 to 6, wherein the plurality of RF passive sensors comprises an array of polyethyleneimine based sensor (I31 COM HBPEI-based sensor), linear polyethyleneimine based sensor (I23 LPEI-COPh-based sensor), polyethyleneimine silane-based sensor (I33 HBPEI-Silane-based sensor), linear polyethyleneimine CH₂-Ph based sensor (I34 LPEI-CH₂-Ph based sensor).

8. Gas radiofrequency sensing system according to claim 7, wherein the I31 COM HBPEI-based sensor is used as a relative humidity "RH" sensor and the I23 LPEI-COPh-based sensor is used as a temperature sensor.

9. Gas radiofrequency sensing system according to any of claims 1 to 8, wherein the plurality of signals comprises a frequency information, a phase information and a magnitude information.

10. Gas radiofrequency sensing system according to any of claims 1 to 9 wherein the at least one interferent parameter includes at least one of a temperature, humidity, wind speed, pressure.

11. Gas radiofrequency sensing system according to any of claims 1 to 10, wherein the at least one target gases and vapors is chosen in the group comprising: NO₂, O₃, CO, VOC, CO₂, NO, H₂S, SO₂, NH₃, O₂, Cl₂, H₂, liquefied petroleum gas, Acetylene, polycyclic aromatic hydrocarbons (PAHs), aromatic hydrocarbons: benzene, toluene, xylene, ethyl benzene; halogenated hydrocarbons: chloroethylene, trichloroethylene; propane, butane, methane methyl chloride; formaldehyde, toluene, acetone, isopropyl alcohol; pesticides: chlordane, DDT; plasticizers: phthalates ;Refrigerant gases: hydrofluorocarbons, chlorofluorocarbons, hydrochlorofluorocarbons.

12. Gas radiofrequency sensing system according to any of claims 1 to 11, wherein each of RF passive sensor is configured to generate a signal within a microwave frequency range between 0.3 and 300 GHz, preferably between 0.5 GHz and 10 GHz.

13. Gas radiofrequency sensing system according to any of claims 1 to 12, further comprising a reader unit (3) configured to generate and transmit a measurement interrogation signal and a reference interrogation signal to each of the gas sensors, receive the measurement signal and the reference signal from each of the gas sensors and transmit the measurement signal and the reference signal to said calculating unit (4).

14. Gas radiofrequency sensing system according to any of the claims 1 to 13, wherein the machine learning model are a K-Nearest Neighbors, a Random Forest, a Gaussian Process Regression models algorithm.

15. Method for operating a gas radiofrequency sensing system according to any of claims 1 to 14, said method comprising a measurement phase comprising:
- generating a plurality of signals within a microwave frequency range by each of the RF passive sensors (301);
- acquiring the plurality of signals by the processor (302);
- inputting in the trained machine learning model the plurality of signals (303);
- analyzing the plurality of signals by the trained machine learning model (304);
- predicting from the trained machine learning model at least one first quantitative value corresponding to a concentration of one target gas or vapor from said mixture of gases and vapors and at least one second quantitative value corresponding to an interferent parameter corresponding to operating conditions of the gas sensing system (305).

16. Method of claim 15, further comprising a training phase performed before the measurement phase, said training phase comprising:
- acquiring a plurality of signals from each of the radiofrequency passive sensors (401);
- acquiring a plurality of reference environmental parameters from a plurality of reference sensors (402), said reference environmental parameters including at least one reference gas and at least one reference interferent parameter;
- inputting in the machine learning model the plurality of signals and the plurality of reference environmental parameters (403);
- training said machine learning model on a calibration model establishing a calibration relationship between each of the signal from the plurality of RF passive sensors and the plurality of reference environmental parameters (404);
- storing said trained machine learning model in the memory (405).
